(19) Europäisches Patentamt

European Patent Office

Office européen des brevets

(11) **EP 3 268 735 B1**

## (12) FASCICULE DE BREVET EUROPEEN

(45) Date de publication et mention de la délivrance du brevet:
**12.11.2025 Bulletin 2025/46**

(21) Numéro de dépôt: **16712966.7**

(22) Date de dépôt: **11.03.2016**

(51) Classification Internationale des Brevets (IPC):
**G01N 33/38** *(2006.01)*    **G01N 22/04** *(2006.01)*

(52) Classification Coopérative des Brevets (CPC):
**G01N 22/04; G01N 33/383**

(86) Numéro de dépôt international:
**PCT/FR2016/050551**

(87) Numéro de publication internationale:
**WO 2016/142634 (15.09.2016 Gazette 2016/37)**

(54) **MESURE NON-DESTRUCTIVE DU TAUX D'HUMIDITÉ D'UN MATÉRIAU**

ZERSTÖRUNGSFREIE MESSUNG DES FEUCHTEGRADS EINES MATERIALS

NON-DESTRUCTIVE MEASUREMENT OF THE DEGREE OF HUMIDITY OF A MATERIAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **11.03.2015 FR 1552032**

(43) Date de publication de la demande:
**17.01.2018 Bulletin 2018/03**

(73) Titulaires:
- **Ecole Supérieure de Physique et de Chimie Industrielles de la Ville de Paris**
  **75005 Paris (FR)**
- **Centre National de la Recherche Scientifique**
  **75794 Paris Cedex 16 (FR)**
- **Sorbonne Université**
  **75006 Paris (FR)**

(72) Inventeurs:
- **MABIRE, Philippe**
  **91080 Courcouronnes (FR)**
- **MAYER, André**
  **77590 Bois Le Roi (FR)**
- **HOLE, Stéphane**
  **75013 Paris (FR)**
- **DITCHI, Thierry**
  **91310 Leuville Sur Orge (FR)**
- **GERON, Emmanuel**
  **45640 Sandillon (FR)**
- **MOKTHARI, Zoé**
  **92130 Issy Les Moulineaux (FR)**

(74) Mandataire: **Plasseraud IP**
**104 Rue de Richelieu**
**CS92104**
**75080 Paris Cedex 02 (FR)**

(56) Documents cités:
WO-A1-2013/088079    DE-U1- 202013 102 514
DE-U1- 202013 102 514    US-A- 3 684 952
US-A- 5 437 181    US-A- 6 111 415

- . ZBYSEK PAVLIK ET AL: "Application of Time-domain Reflectometry Method for Measuring Moisture Content in Porous Building Materials", TRENDS IN APPLIED SCIENCES RESEARCH, vol. 2, no. 3, 1 March 2007 (2007-03-01), pages 188 - 200, XP055796824, ISSN: 1819-3579, DOI: 10.3923/tasr.2007.188.200
- LARSSON O ET AL: "Proton motion in a polyelectrolyte: A probe for wireless humidity sensors", SENSORS AND ACTUATORS B: CHEMICAL, ELSEVIER BV, NL, vol. 143, no. 2, 7 January 2010 (2010-01-07), pages 482 - 486, XP026821465, ISSN: 0925-4005, [retrieved on 20090930]
- CATALDO ANDREA ET AL: "Hydration Monitoring and Moisture Control of Cement-Based Samples Through Embedded Wire-Like Sensing Elements", IEEE SENSORS JOURNAL, IEEE, USA, vol. 15, no. 2, 1 February 2015 (2015-02-01), pages 1208 - 1215, XP011566325, ISSN: 1530-437X, [retrieved on 20141202], DOI: 10.1109/JSEN.2014.2360712

**(Cont. page suivante)**

- X JIN ET AL: "EMBEDDED ANTENNAS IN DRY AND SATURATED CONCRETE FOR APPLICATION IN WIRELESS SEN- SORS", PROGRESS IN ELECTROMAGNETICS RESEARCH, vol. 102, 1 January 2010 (2010-01-01), pages 197 - 211, XP055491842
- RAD M F ET AL: "Embedded microstrip patch antenna for structural health monitoring applications", ANTENNAS AND PROPAGATION SOCIETY INTERNATIONAL SYMPOSIUM, 2008. AP-S 2008. IEEE, IEEE, PISCATAWAY, NJ, USA, 5 July 2008 (2008-07-05), pages 1 - 4, XP031824790, ISBN: 978-1-4244-2041-4
- SOONTORNPIPIT P ET AL: "Optimization of a buried microstrip antenna for simultaneous communication and sensing of soil moisture", IEEE TRANSACTIONS ON ANTENNAS AND PROPAGATION, IEEE, USA, vol. 54, no. 3, 1 March 2006 (2006-03-01), pages 797 - 800, XP001545332, ISSN: 0018-926X, DOI: 10.1109/ TAP.2006.869904
- SAN-SHAN HUNG ET AL: "Packaged wireless multisensor module embedded reinforced concrete for monitoring construction characteristics", ELECTRONIC PACKAGING TECHNOLOGY&HIGH DENSITY PACKAGING (ICEPT-HDP), 2010 11TH INTERNATIONAL CONFERENCE ON, IEEE, PISCATAWAY, NJ, USA, 16 August 2010 (2010-08-16), pages 1293 - 1296, XP031761154, ISBN: 978-1-4244-8140-8
- KUPFER K: "Radiofrequency and Microwave Moisture Sensing of Building Materials", SENSORS UPDATE, XX, XX, vol. 7, 1 January 2000 (2000-01-01), pages 27 - 50, XP002326260, DOI: 10.1002/1616-8984(200001)7:1<27::AID-SEUP27>3.0.CO;2-G

**Description**

*Domaine technique et Art antérieur*

La présente invention a trait au domaine des mesures, et concerne plus particulièrement les mesures dites non-destructives pour mesurer et suivre l'évolution du taux d'humidité d'un matériau.

L'objet de la présente invention porte ainsi sur une technique de mesure non destructive permettant de mesurer, avec précision et à moindre coût, le taux d'humidité d'un matériau.

La présente invention trouve de nombreuses applications avantageuses, notamment dans l'industrie de la construction, et particulièrement l'industrie du bâtiment, en permettant de déterminer le taux d'humidité d'un matériau solide comprenant un ou plusieurs liants minéraux tel que par exemple le mortier ou le béton.

On peut citer comme exemple d'applications de la présente invention la construction d'ouvrages d'art (pont, barrage, etc.) ou de bâtiments (ou locaux) à usage d'habitation ou professionnel, ainsi que l'aménagement ou la réhabilitation desdits bâtiments, comprenant la pose de dalles de béton ou de chapes de mortier ou encore de ragréages.

D'autres applications avantageuses peuvent également être envisagées dans le cadre de la présente invention, comme par exemple la mesure et le suivi du taux d'humidité :

- d'un sol naturel (composé par exemple de limon, de sable, d'argile, etc.),
- d'un matériau solide exempt de liant minéral et comprenant un ou plusieurs liants organiques (par exemple un corps gélatineux, un dérivé d'amidon, etc.),
- d'un matériau solide composite obtenu par frittage (par exemple un objet en céramique),
- d'un matériau pulvérulent (par exemple une farine, de la sciure de bois, etc.),
- de bois sous la forme par exemple de copeaux, de granulés ou de plaquettes, ou encore
- d'un matériau liquide.

Par taux d'humidité du matériau au sens de la présente invention, il faut comprendre ici dans toute la présente description qui suit la teneur en eau du matériau, c'est-à-dire la quantité d'eau contenue dans le matériau.

Le béton est un matériau de construction composite fabriqué à partir notamment de granulats (graviers de taille supérieure à 1 cm) agglomérés par un liant minéral pulvérulent (par exemple un ciment, de la chaux ou du sulfate de calcium).

Le mortier est également un matériau de construction composite fabriqué à partir notamment de granulats (gravillons de taille inférieure à 1 cm) agglomérés par un liant minéral pulvérulent semblable à celui d'un béton.

Généralement, qu'il s'agisse d'un béton ou d'un mortier, le liant minéral est hydraté, autrement dit mis en contact avec de l'eau au cours d'un processus de mélange dénommé « gâchage », pour permettre l'agglomération des granulats.

On parle de béton (ou mortier) à base de liants hydrauliques.

Généralement, au cours des processus physiques et chimiques qui suivent la mise en contact du matériau (béton ou mortier) avec l'eau de gâchage, une partie de cette eau reste liée aux cristaux formés à partir du liant minéral. On parle d'eau de cristallisation.

Une autre partie de l'eau, dite résiduelle, reste à l'état libre dans la porosité du béton (ou du mortier) ; cette eau résiduelle s'évapore au moins partiellement avec le temps, laissant place à un matériau solide.

Dans le domaine du bâtiment, certains ouvrages en béton (notamment les dalles de béton) ou en mortier (notamment les chapes ou couches de ragréage) sont souvent appelés à recevoir un produit de recouvrement tel qu'une couche de résine synthétique, une couche de peinture, ou encore une couche d'un revêtement posé ou collé (un parquet en bois, un carrelage ou encore un sol en plastique).

Avant de procéder au recouvrement du béton (par exemple sous la forme de dalle) ou du mortier (par exemple sous la forme d'une chape ou d'une couche de ragréage), il est impératif de s'assurer que le taux d'humidité résiduelle du matériau solide (béton ou mortier) soit suffisamment bas pour garantir une bonne tenue.

Une phase de séchage est donc nécessaire ; celle-ci peut prendre plusieurs jours, voire plusieurs semaines.

On note ici que le taux d'humidité résiduelle dans le béton (ou mortier) correspond au rapport entre le poids de l'eau libre et le poids du béton (ou mortier) sec.

Ce taux d'humidité résiduelle s'exprime usuellement en pourcentage ; on parle aussi de taux de siccité.

L'eau libre contenue dans le béton (ou mortier) s'évapore progressivement lors du séchage ; le taux d'humidité résiduelle va donc évoluer au cours de la phase de séchage.

Ainsi, lorsque ce taux d'humidité est satisfaisant, il est possible de poursuivre l'ouvrage et d'appliquer par exemple sur la dalle de béton ou la chape de mortier (ou la couche de ragréage) les couches supérieures.

On comprend ici que, dans l'industrie de la construction, notamment du bâtiment, connaître le taux d'humidité d'un béton ou d'un mortier en cours de séchage est essentiel pour déterminer le moment le plus approprié pour poursuivre l'ouvrage ; connaître avec précision ce taux d'humidité permet d'optimiser les temps de construction tout en garantissant la solidité et les propriétés relatives à la résistance mécanique de l'ouvrage.

Les techniques développées jusqu'à présent pour déterminer avec précision le taux d'humidité à l'intérieur du béton ou du mortier sont bien souvent des techniques de mesures dites destructives.

Ces techniques destructives nécessitent une destruction partielle du matériau sur lequel il faut réaliser la mesure ; elles requièrent un prélèvement d'échantillon.

L'opérateur en charge sur le chantier de contrôler le taux d'humidité du matériau solide (béton ou mortier) prélève à cœur un échantillon dudit matériau.

Ensuite, il réduit en poudre cet échantillon, puis le fait interagir avec par exemple du carbure de calcium dans une enceinte hermétique (bombe à carbure de calcium).

Ceci provoque une réaction chimique entre le carbure et l'eau contenue dans l'échantillon de matériau testé.

Cette réaction entraîne un dégagement de gaz qui augmente la pression dans l'enceinte hermétique.

Il existe donc un lien direct entre la quantité d'eau contenue dans l'échantillon de béton (ou de mortier) prélevé et la variation de pression sous l'enceinte.

Pour déterminer le taux d'humidité du béton (ou du mortier), l'opérateur mesure donc la variation de pression dans l'enceinte avec un baromètre ; de préférence, ce baromètre est intégré directement dans l'enceinte.

Il existe d'autres techniques de mesures destructives.

Suite à un prélèvement, il est possible alternativement de sécher en étuve l'échantillon prélevé (par exemple à 110°C), et ensuite de comparer le poids de l'échantillon avant et après séchage.

En fonction de cette comparaison, il est possible de déterminer la quantité d'eau contenue dans le béton (ou dans le mortier) avant séchage.

Ces différentes techniques sont destructives.

Bien que tolérable, le prélèvement d'un échantillon, nécessaire pour réaliser la mesure, n'est pas satisfaisant (surtout dans l'industrie de la construction et notamment celle du bâtiment).

Par ailleurs, il n'est pas vraiment possible avec ces techniques de tester l'homogénéité du séchage ; en effet, cela nécessiterait la réalisation de nombreux prélèvements dans l'ouvrage, par exemple une dalle en cours de séchage, ce qui entraînerait inévitablement plus de destructions.

Ces techniques destructives nécessitent de plus l'intervention relativement longue d'un technicien qualifié.

Dans ces conditions, il est difficile en utilisant ces techniques de suivre finement l'évolution du taux d'humidité d'un matériau tel que le béton ou le mortier : ces techniques sont en effet longues, complexes et fastidieuses à mettre en œuvre ; elles sont peu pratiquées sur chantier.

Il existe des techniques de mesures non-destructives permettant d'estimer le taux d'humidité d'un matériau.

Cependant, les techniques de mesures non-destructives développées jusqu'à présent ne sont pas entièrement satisfaisantes.

Le taux d'humidité d'un béton ou d'un mortier en cours de séchage peut en effet être mesuré en surface par un appareil du type humidimètre à pointe.

Un tel appareil utilise les propriétés relatives à la conductivité électrique du matériau testé.

On sait en effet que la conductivité électrique du béton ou du mortier varie en fonction de la teneur en eau : la présence d'eau dans le matériau favorise la conductivité de l'électricité dans le matériau.

Selon cette technique, une tension est donc appliquée entre deux voire plusieurs pointes, ou électrodes, mises en contact avec le béton ou le mortier.

La résistance électrique mesurée entre les pointes donne alors une information sur la quantité d'eau dans le béton ou le mortier.

Avec cette technique, on observe toutefois des erreurs dans les mesures réalisées.

Ces erreurs proviennent principalement de réactions chimiques entre les électrodes et le béton ou le mortier.

On observe également un changement de percolation entre les grains du béton ou du mortier.

De plus, cette technique n'est pas satisfaisante ; en effet, le taux d'humidité mesuré est celui en surface.

Or, la surface du béton ou du mortier sèche plus rapidement que le cœur.

La surface du matériau présente en outre des polluants comme de la poussière ainsi que des irrégularités qui peuvent fausser la mesure.

Le taux d'humidité qu'il est pertinent de prendre en considération pour poursuivre l'ouvrage est donc celui à l'intérieur du béton (ou du mortier) ; c'est-à-dire celui à cœur.

Pour réaliser ces mesures à cœur, d'autres techniques existent ; elles sont toutefois plus complexes, et nécessitent l'intégration de capteurs dans le matériau.

Dans ces techniques, c'est bien souvent la permittivité diélectrique du matériau à tester qui est le paramètre physique retenu pour réaliser la mesure.

En effet, cette constante diélectrique est de l'ordre de quelques unités pour la plupart des matériaux ; elle est de l'ordre de 80 pour l'eau.

Par conséquent, la présence d'eau dans un matériau tel que le béton ou le mortier augmente singulièrement la constante diélectrique mesurée.

Les lois de l'électromagnétisme sont alors utilisées pour faire la mesure, soit par effet capacitif à basse fréquence, soit avec une cavité résonante à haute fréquence.

L'inconvénient majeur des techniques utilisées est l'encombrement des capteurs utilisés ou leur faible rayon d'action lorsque ces capteurs sont miniaturisés.

En effet, comme la mesure doit être faite au cœur du matériau testé, un grand encombrement modifie nécessairement la façon dont le matériau sèche : la mesure réalisée n'est donc pas fiable.

Lorsque le capteur est miniaturisé, le séchage n'est pas perturbé mais des incertitudes de mesure apparaissent de par l'aspect granulaire du béton ou du mortier : le rayon d'action d'un capteur miniaturisé est nécessairement petit.

La réponse du capteur diffère donc en fonction de son environnement ; la réponse est différente selon qu'il est proche d'un constituant ou d'un autre du béton ou du mortier.

Les techniques de mesures non-destructives ne sont donc pas satisfaisantes, notamment pour des raisons de coûts (capteurs) et pour des raisons de précision.

DE 20 2013 102 514 U1 divulgue une sonde émettant un signal micro-ondes au sein d'un matériau et mesurant le temps de vol du signal pour estimer la constante diélectrique du matériau. La sonde ne constitue pas une antenne et l'estimation de la constante diélectrique ne comporte aucune analyse fréquentielle.

Des exemples et des modes de réalisation possibles de l'art antérieur peuvent être trouvés dans les documents KUPFER K : « Radiofrequency and Microwave Moisture Sensing of Building Materials », SENSORS UPDATE, vol. 7, 1 janvier 2000, pages 27-50; Zbysek Pavlik ET Al : « Application of Time-domain Reflectometry Method for measuring moisture content in porous building materials », Trends in Applied Sciences Research, vol. 2, no. 3, 1 mars 2007, pages 188-200; CATALDO ANDREA ET AL: "Hydration Monitoring and Moisture Control of Cement-Based Samples Through Embedded Wire-Like Sensing Elements", IEEE SENSORS JOURNAL, IEEE, USA, vol. 15, no. 2, 1 février 2015, pages 1208-1215; et SOONTORNPIPIT P ET AL: "Optimization of a buried microstrip antenna for simultaneous communication and sensing of soil moisture", IEEE TRANSACTIONS ON ANTENNAS AND PROPAGATION, IEEE, USA, vol. 54, no. 3, 1 mars 2006, pages 797-800

Compte tenu des inconvénients mentionnés ci-dessus pour les différentes techniques déployées jusqu'à présent, l'industrie de la construction, notamment celle du bâtiment, préfère augmenter la durée globale de la construction d'un ouvrage en laissant une marge de sécurité dans les temps de séchage.

Ceci engendre cependant des coûts indirects (durée générale du chantier, report de livraison, etc.).

*Objet et résumé de la présente invention*

La présente invention vise à améliorer la situation décrite ci-dessus.

Un des objectifs de la présente invention est de remédier aux différents inconvénients mentionnés ci-dessus en proposant une solution non-destructive, simple et peu coûteuse permettant de mesurer et de suivre avec précision le taux d'humidité à cœur d'un matériau, en particulier d'un matériau solide comme par exemple le béton, le mortier ou encore d'autres matériaux.

À cet effet, l'objet de la présente invention concerne selon un premier aspect un procédé de mesure non-destructive pour mesurer un taux d'humidité d'un matériau, selon la revendication 1.

Ainsi, grâce à cette succession d'étapes techniques, caractéristique de la présente invention, il suffit à la personne en charge de mesurer le taux d'humidité d'un matériau de faire coopérer avec l'antenne un appareil de mesure du type réflectomètre, de préférence portatif.

Une partie du signal radiofréquence émis est alors réfléchie vers l'appareil de mesure, tandis qu'une autre partie de ce signal est transmise dans l'environnement de l'antenne, c'est-à-dire ici dans le matériau.

Selon la fréquence du signal, l'efficacité de la transmission change.

Il existe donc des fréquences pour lesquelles la transmission est plus efficace.

Ces fréquences dépendent de la géométrie de l'antenne mais également de la constante diélectrique (permittivité relative) du matériau à proximité de l'antenne (par exemple le béton ou le mortier avec tous ses constituants dont l'eau).

Ainsi, les fréquences de transmission sont directement liées à la teneur en eau du matériau, mais également au comportement de l'eau à la fréquence des mesures.

Il est alors possible de discerner les différents types de liaisons des molécules d'eau avec le matériau, et donc d'analyser plus finement le taux d'humidité et l'état d'avancement du séchage.

L'appareil de mesure reçoit donc, en réponse au signal émis, un signal réfléchi par l'antenne.

Ce signal réfléchi contient toutes les informations relatives à cette teneur en eau : ce qui n'est pas transmis dans le matériau est réfléchi par l'antenne.

Il apparait donc un pic négatif dans le signal réfléchi aux fréquences où le signal est transmis.

L'atténuation que présente le signal réfléchi par l'antenne par rapport au signal radiofréquence émis s'exprime de façon générale comme un nombre complexe. L'analyse en fréquence peut s'effectuer pour un déphasage nul entre le signal réfléchi et le signal émis, ce qui correspond à un cas usuel d'utilisation d'un réflectomètre. On observera qu'il est aussi possible d'avoir un déphasage non nul entre le signal réfléchi et le signal émis, ce qui décale les fréquences de résonance déterminées. Un tel décalage des fréquences de résonance n'empêche pas l'interprétation des mesures pour l'estimation du taux d'humidité.

Dans un mode de réalisation simple, le signal réfléchi par l'antenne est donc reçu et analysé en fréquence avec un déphasage nul par rapport au signal émis.

Avantageusement, l'analyse en fréquence du signal réfléchi par l'antenne comprend un calcul de coefficients de réflexion du signal réfléchi pour déterminer les fréquences de résonance.

Avantageusement, le procédé selon la présente invention comporte une étape finale au cours de laquelle, lorsque le taux d'humidité mesuré est égal ou supérieur à un taux d'humidité seuil prédéterminé, l'interface entre l'antenne et l'appareil de mesure, si elle est de type filaire, est sectionnée, de préférence au ras du matériau.

Dans une application pour l'industrie de la construction, notamment du bâtiment, une fois que la teneur en eau visée pour le béton (ou pour le mortier) est atteinte, l'interface dépassant le cas échéant du béton (ou du mortier) peut être coupée pour passer à l'étape suivante dans la réalisation de l'ouvrage.

L'antenne est alors perdue dans le matériau (par exemple dans le béton ou dans le mortier).

Compte tenu du coût d'une telle antenne, ceci est tout à fait acceptable par rapport aux solutions existantes décrites précédemment.

Le procédé de mesure proposé dans le cadre de la présente invention est donc simple et rapide à mettre en œuvre ; la personne en charge de la mesure du taux d'humidité d'un matériau tel que par exemple le béton ou le mortier n'a pas besoin d'avoir de qualification particulière car il lui suffit de faire coopérer l'appareil de mesure avec l'antenne, puis de lire le résultat affiché sur l'appareil.

On comprend ainsi que le procédé de mesure proposé dans le cadre de la présente invention est particulièrement intéressant pour l'industrie de la construction (et notamment l'industrie du bâtiment) : il permet à l'opérateur de mesurer, rapidement et plusieurs fois si nécessaire, le taux d'humidité d'un matériau comme le béton ou le mortier ; un tel procédé lui permet notamment de gérer efficacement les temps de séchage et de décider ou non la poursuite de l'ouvrage.

L'objet de la présente invention concerne, selon un deuxième aspect, une utilisation d'un procédé de mesure non-destructive tel que décrit ci-dessus pour mesurer le taux d'humidité d'un matériau solide comprenant un ou plusieurs liants minéraux.

Avantageusement, les liants minéraux sont notamment choisis parmi un ciment, de la chaux ou du sulfate de calcium.

Le matériau solide visé par ladite utilisation est de façon particulièrement avantageuse un béton ou un mortier en cours de séchage.

Avantageusement, lorsque le matériau est un mortier en cours de séchage, les fréquences de résonance supérieures ou égales à 1,8 GHz sont considérées comme caractérisant une présence d'eau de cristallisation dans le mortier, tandis que les fréquences de résonance strictement inférieures à 1,8 GHz sont considérées comme caractérisant une présence d'eau résiduelle dans le mortier.

On préfère, selon une variante tout particulièrement préférée de cette même utilisation, mettre en œuvre ledit matériau solide comme élément de la structure de sol d'un local à usage d'habitation ou professionnel, pour la pose d'une couche de revêtement de sol sur ladite structure.

On entend désigner par structure de sol la structure comprenant la dalle de béton qui résulte de l'ouvrage de gros œuvre correspondant à la construction du bâtiment, ladite dalle étant de préférence recouverte d'une chape de mortier et/ou d'une couche de ragréage obtenues par coulage de mortier liquide. La couche de ragréage a pour but de niveler les défauts de surface de la couche sous-jacente (dalle de béton ou chape de mortier), afin d'obtenir une surface plane et lisse, propre à recevoir un revêtement de sol.

Parmi les revêtements de sol, qui peuvent être posés, de préférence par collage, sur ladite structure, on peut citer par exemple, et de manière non limitative, un parquet, un carrelage, un revêtement souple comme de la moquette tricotée, touffetée, tissée, floquée, en lés ou en dalles, un revêtement de sol aiguilleté, en lés ou en dalles, un revêtement de sol homogène ou hétérogène à base de polychlorure de vinyle, un revêtement de sol à base de polychlorure de vinyle sur support de jute ou de polyester ou sur support de polyester avec envers en polychlorure de vinyle, un revêtement de sol à base de polychlorure de vinyle sur mousse, un revêtement de sol à base de polychlorure de vinyle avec support à base de liège, un revêtement de sol à base de polychlorure de vinyle expansé, une dalle semi flexible à base de polychlorure de vinyle ou encore une dalle d'aggloméré de liège avec couche d'usure à base de polychlorure de vinyle.

Selon cette dernière variante de réalisation, la mise en œuvre du matériau solide sous la forme d'une dalle de béton ou d'une chape de mortier, ou encore d'une couche de ragréage, est particulièrement avantageuse.

L'objet de la présente invention concerne, selon un troisième aspect, une utilisation d'un procédé de mesure non-destructive tel que décrit ci-dessus pour mesurer le taux d'humidité d'un matériau choisi parmi :

- un sol naturel,
- un matériau solide exempt de liant minéral et comprenant un ou plusieurs liants organiques,
- un matériau solide composite obtenu par frittage,
- un matériau pulvérulent,
- du bois, par exemple sous la forme de copeaux, de sciure, de granulés ou de plaquettes, ou
- un matériau liquide.

Comme évoqué en préambule de la présente description, d'autres utilisations du procédé selon l'invention peuvent également être envisagées dans le cadre de la présente invention.

Corrélativement, l'objet de la présente invention concerne, selon un quatrième aspect, un système de mesure non-destructive pour mesurer un taux d'humidité d'un matériau, selon la revendication 10.

Plus particulièrement, le système selon la présente invention comporte :

- un appareil de mesure du type réflectomètre ;
- une antenne noyée dans le matériau, et
- une interface entre l'antenne et l'appareil de mesure.

L'appareil de mesure du système est configuré pour :

- émettre un signal radiofréquence dans le matériau à travers l'antenne ;
- recevoir un signal réfléchi par l'antenne en réponse au signal radiofréquence émis ;
- analyser en fréquence le signal réfléchi pour déterminer des fréquences de résonance de l'antenne dans le matériau ;
- estimer le taux d'humidité du matériau à partir des fréquences de résonance déterminées.

L'antenne est du type dipolaire.
Dans un mode de réalisation avantageux, l'antenne est de type filaire et comprend deux segments qui s'étendent chacun dans le matériau entre une extrémité proximale et une extrémité distale par rapport à l'interface avec l'appareil de mesure, les deux segments définissant des vecteurs ayant entre eux un produit scalaire négatif.
Les deux segments forment ainsi une antenne du type lambda sur deux.
Dans une variante, les segments sont coplanaires.
De préférence, ce plan est le plan médian ; ce plan médian correspond ici au plan qui s'étend dans la partie médiane du matériau (c'est-à-dire le plan s'étendant dans la couche centrale du matériau, à cœur).
Dans une autre variante, les segments sont chacun respectivement dans des plans qui s'étendent longitudinalement dans le sens du matériau.
De la même façon que précédemment, chacun de ces plans s'étend sensiblement dans la partie médiane du matériau.
Dans un cas comme dans l'autre, le fait que les segments s'étendent dans la partie médiane du matériau permet de réaliser la mesure au plus près du cœur du matériau.
De préférence, les segments de l'antenne présentent chacun des dimensions identiques (longueur, diamètre).
Avantageusement, les deux segments s'étendent dans des directions sensiblement opposées. En outre, ils peuvent chacun être d'une longueur comprise entre 5 à 15 centimètres, de préférence sensiblement égale à 7,5 centimètres.
Avantageusement, l'antenne est isolée du matériau par une gaine protectrice, de préférence en résine époxyde.
Il est préférable en effet que l'antenne soit isolée par une résine neutre de sorte à la rendre insensible à son environnement, par exemple le béton (ou le mortier) et toutes les réactions chimiques qui y ont lieu.

Avantageusement, l'interface entre l'antenne et l'appareil de mesure, si elle est de type filaire, comporte une connectique configurée pour connecter à l'appareil de mesure du type réflectomètre.
De préférence, l'interface est alors configurée pour pouvoir être retirée, par exemple par l'intermédiaire d'une section sécable sous forme de prédécoupe ou d'amincissement de section.
Avantageusement, il est possible de prévoir que le système comporte un flotteur amovible apte à être fixé temporairement à la l'interface dépassant du matériau ; ce flotteur est configuré pour régler le positionnement de l'antenne dans le matériau, par exemple lors du coulage du matériau et/ou lorsque celui-ci est à l'état liquide.
Un tel flotteur est particulièrement avantageux pour positionner correctement l'antenne par rapport au matériau à tester, notamment pour la positionner pendant le coulage du béton (ou du mortier) frais sur un sol horizontal, par exemple lors du coulage d'une dalle de béton, d'une chape de mortier, ou encore d'une couche de ragréage.
Ainsi, la présente invention, par ses différentes caractéristiques techniques structurelles et fonctionnelles, permet de mesurer simplement et avec précision le taux d'humidité d'un matériau tel que par exemple un béton ou un mortier en cours de séchage.
La mesure est non-destructive et se fait à cœur par la présence d'un capteur peu coûteux.

*Brève description des figures annexées*

D'autres caractéristiques et avantages de la présente invention ressortiront de la description ci-dessous, en référence aux figures 1 à 6 annexées qui en illustrent un exemple de réalisation dépourvu de tout caractère limitatif et sur lesquelles :

- la figure 1 représente une vue schématique d'un système selon un exemple de réalisation avec un appareil de mesure du type réflectomètre connecté à une antenne de type dipolaire noyée dans une chape de mortier ;
- la figure 2 représente une vue en coupe d'une section d'interface entre l'antenne et l'appareil de mesure ;
- les figures 3a à 3h sont des graphiques, issus de résultats expérimentaux, représentant chacun l'évolution des coefficients de réflexion du signal réfléchi en fonction d'une fréquence allant de 100 MHz à 3GHz, ceci à différents instants pendant la phase de séchage du mortier ;
- les figures 4a, 4b et 4c sont des graphiques représentant l'évolution pendant les 30 premiers jours de séchage de la fréquence de chacun des pics fréquentiels observés pour les coefficients de réflexion ;
- les figures 5a, 5b et 5c sont des graphiques représentent l'évolution de la fréquence de chacun des trois pics observés et l'évolution de la masse du mortier pendant les premières heures de séchage ;

- la figure 6 est un organigramme illustrant le procédé selon un exemple de mise en œuvre.

*Description détaillée d'un exemple de réalisation avantageux*

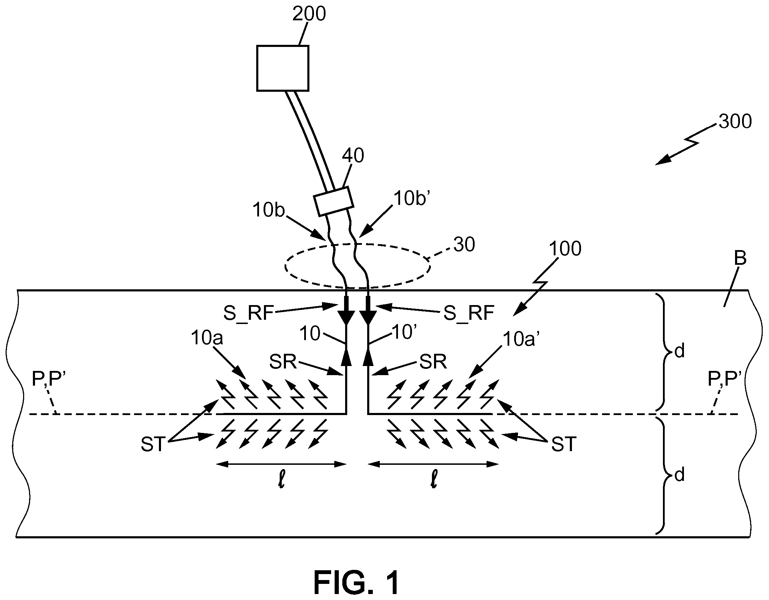

Une mesure non-destructive selon un exemple de réalisation avantageux ainsi que le système associé vont maintenant être décrits dans ce qui va suivre en faisant référence conjointement aux figures 1 à 6.

L'exemple décrit ici concerne le suivi du taux d'humidité d'un mortier sous forme de chape.

On comprendra qu'il s'agit d'un exemple purement illustratif ne présentant en aucun cas un caractère limitatif.

En effet, comme énoncé précédemment, d'autres exemples de mise en œuvre pour des matériaux autres que le mortier peuvent être envisagés dans le cadre de la présente invention.

On sait qu'une phase de séchage est nécessaire pour qu'une chape de mortier atteigne des caractéristiques mécaniques compatibles avec les normes de la construction, notamment pour pouvoir poser une couche de revêtement sur ladite chape.

Jusqu'à présent, cette phase est contrôlée par des mesures complexes effectuées sur des prélèvements d'échantillons : par exemple une mesure destructive du type chimique utilisant du carbure de calcium ou une mesure de poids.

Dans l'exemple décrit ici, concevoir un système évitant ces prélèvements et permettant une mesure précise, peu coûteuse et simple à réaliser, afin de déterminer le taux d'humidité d'un mortier en cours de séchage est un des objectifs de la présente invention.

D'une part, cette mesure doit être non-destructive, notamment pour satisfaire aux exigences de l'industrie du bâtiment.

D'autre part, cette mesure doit se faire à cœur, et non en surface, pour être la plus précise.

Ceci est rendu possible dans le cadre de la présente invention par la conception d'un capteur 100, appelé encore capteur perdu.

Comme évoqué précédemment, pour déterminer les propriétés diélectriques d'un matériau comme le mortier et donc connaître son taux d'humidité, il est possible de réaliser des mesures à basse fréquence, par exemple en réalisant des mesures par effet capacitif. Ceci n'est pas très satisfaisant.

Le concept sous-jacent à la présente invention repose, lui, sur une mesure à haute fréquence utilisant une antenne hyperfréquence, de type dipolaire.

Dans l'exemple décrit ici, le capteur utilisé 100 comporte une antenne composée de deux fils 10 et 10'.

Lors d'une étape préalable S0, ce capteur 100 est inséré partiellement dans la chape de mortier B au cours de la pose, ici au cours du coulage de ladite chape.

Suite à cette étape S0, les deux fils 10-10' de l'antenne présentent chacun :

- deux segments 10a-10a' qui forment l'antenne proprement dite, qui sont noyés dans le mortier B, et
- une interface 10b-10b', 40 entre l'antenne (les deux segments 10a-10a') et l'appareil de mesure 200, l'interface dépassant ici du mortier B.

Dans l'exemple décrit ici, les segments 10a et 10a' s'étendent chacun horizontalement respectivement selon des plans P et P'.

Ces plans P et P' sont sensiblement parallèles entre eux.

Plus particulièrement, dans cet exemple, les plans P et P' sont confondus : les premières portions 10a et 10a' sont donc coplanaires.

Les segments peuvent être disposés selon d'autres agencements. En particulier, les segments qui s'étendent chacun dans le matériau B peuvent être disposés de telle sorte que des vecteurs définis entre une extrémité proximale et une extrémité distale des deux segments par rapport à l'interface avec l'appareil de mesure, définissent des vecteurs ayant entre eux un produit scalaire négatif, c'est-à-dire qu'ils ont entre eux un angle compris entre $\pi/2$ et $3\pi/2$.

Dans l'exemple décrit ici, les segments 10a et 10a' s'étendent dans la partie médiane de la chape de mortier B de sorte à permettre une mesure à cœur.

Dans cet exemple, l'interface 10b-10b', 40 entre l'antenne et l'appareil de mesure 200 comprend une connectique 40 spécifique qui permet de connecter les segments 10a, 10a' de l'antenne au réflectomètre 200 via les fils de connexion 10b, 10b'.

Plus particulièrement, dans l'exemple décrit ici, la connectique 40 est configurée pour permettre au réflectomètre 200 d'être branché par l'intermédiaire d'un câble coaxial, l'un étant connecté à l'âme centrale des fils 10, 10' et l'autre à la masse des fils.

Selon d'autres réalisations possibles, l'interface entre l'antenne et l'appareil de mesure peut être réalisée sans fil, par exemple via une communication en champ proche (NFC) entre l'antenne et l'appareil de mesure.

Pour favoriser le positionnement de l'antenne dans le mortier B, lorsque cette antenne est de type filaire avec une interface également filaire, il est prévu selon l'invention d'utiliser un flotteur 30.

Plus particulièrement, ce flotteur 30 permet de régler la hauteur relative des segments 10a et 10a' de l'antenne dans le mortier B.

Dans cet exemple, le flotteur 30 est amovible et se fixe sur l'interface 10b- 10b'. Le flotteur 30 permet la mise en place et le positionnement des fils 10 et 10' dans la chape de mortier B lors du coulage.

Une fois les fils 10 et 10' positionnés, on retire le flotteur 30.

Dans l'exemple décrit ici, les segments 10a et 10a' partent chacun dans des directions opposées de manière à former une antenne dipolaire de type lambda sur deux.

Il est préférable que ces fils 10 et 10' soient isolés.

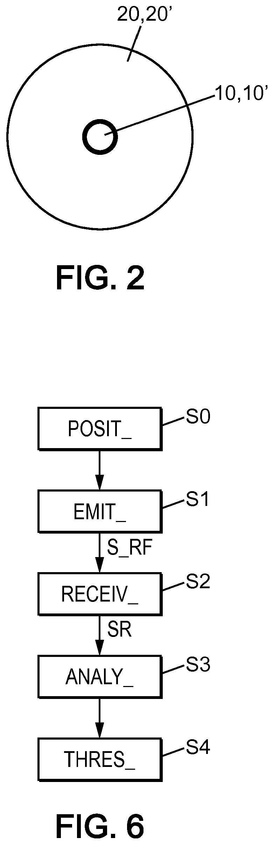

Dans l'exemple décrit ici et illustré en figure 2, il est prévu une gaine protectrice 20 et 20' pour chacun des fils 10 et 10'.

Cette gaine 20, 20' est composée au moins partiellement dans une résine neutre de sorte à ce que les fils 10 et 10' soient insensibles au mortier B et à toutes les réactions chimiques qui y ont lieu.

Le principe sur lequel repose la présente invention est d'envoyer de l'énergie électromagnétique dans les fils 10 et 10', et de mesurer les pertes par rayonnement de ces fils 10 et 10'.

Au centre, les deux fils 10 et 10' (à l'interface 10b--10b') sortent donc du mortier B de manière à pouvoir être connectés avec l'appareil de mesure portatif approprié 200 ; cet appareil 200 est du type réflectomètre.

Dans l'exemple décrit ici, une fois le réflectomètre 200 branché au capteur 100, l'opérateur envoie dans le capteur 100 un signal radiofréquence S_RF depuis le réflectomètre 200, ceci lors d'une étape d'émission S1.

Une partie de ce signal émis S_RF est alors réfléchie vers l'appareil de mesure 200 (on parle de signal réfléchi, noté SR), tandis qu'une autre partie de ce signal S_RF est transmise dans l'environnement du capteur 100, notamment dans le mortier B (on parle de signal transmis, noté ST).

L'appareil de mesure 200 reçoit alors ce signal réfléchi SR par l'antenne lors d'une étape S2.

La fréquence à laquelle l'énergie émise par le signal S_RF est la mieux transmise dans le mortier B dépend des propriétés électromagnétiques (notamment de la permittivité relative) du mortier B au voisinage du dipôle 100.

Cette fréquence dépend directement de la quantité d'eau contenue dans le mortier B.

En effet, comme mentionné précédemment, les fréquences du signal transmis ST sont directement liées à la teneur en eau du mortier B, mais également au comportement de l'eau à la fréquence des mesures.

La corrélation entre la fréquence de résonance et le taux d'humidité d'un matériau tel que le mortier se déduit des formules suivantes :

On sait tout d'abord que la fréquence de résonance est fonction de la vitesse de propagation $v\varphi$ des ondes électromagnétiques et de la longueur L de l'antenne constituée par les segments 10a et 10a' :

$$Fréquence\_résonance = \frac{v\varphi}{2L}$$

La vitesse de propagation $v\varphi$ des ondes électromagnétiques dans le matériau est donnée par :

$$v\varphi = \frac{c}{\sqrt{\varepsilon_r}}$$

dans laquelle la variable $\varepsilon_r$ est la permittivité relative du mortier.

La permittivité relative du mortier sec est de l'ordre de 2 à 7, tandis que la permittivité relative de l'eau est d'environ 80.

Des deux formules ci-dessus, on a la relation suivante entre la fréquence de résonance et le taux d'humidité du mortier :

$$Fréquence\_résonance = \frac{c}{2L\sqrt{\varepsilon_r}}$$

A partir de la fréquence de résonance mesurée et des formules précitées, il est possible de calculer $\varepsilon_r$ qui est la permittivité relative du mortier dans laquelle est noyée l'antenne. De manière connue en soit, la permittivité diélectrique d'un matériau permet d'établir le taux d'humidité de ce matériau. De ce fait, la permittivité calculée $\varepsilon_r$ selon la fréquence de résonance mesurée permet de déterminer le taux d'humidité du mortier testé.

Le signal réfléchi SR par l'antenne, accessible directement à partir de l'appareil de mesure 200, contient donc toutes les informations relatives à la teneur en eau du mortier B : en effet, si le signal est transmis par les fils 10, 10' vers le mortier B, celui-ci n'est pas réfléchi vers l'appareil 200.

Le signal réfléchi par l'antenne peut notamment être reçu et analysé en fréquence avec un déphasage nul par rapport au signal émis.

Il suffit alors de calculer les coefficients de réflexion propre au signal réfléchi pour obtenir les fréquences de résonance.

Dans l'exemple décrit ici, la réponse en fréquence est enregistrée au cours du temps par un balayage en fréquence entre 100MHz et 3 GHz, avec le réflectomètre 200 ou un analyseur de réseau.

Suite à cette mesure, on calcule l'évolution du coefficient de réflexion du signal SR au cours du balayage (i.e. en fonction de la variation de fréquence), ceci à plusieurs instants pendant le séchage.

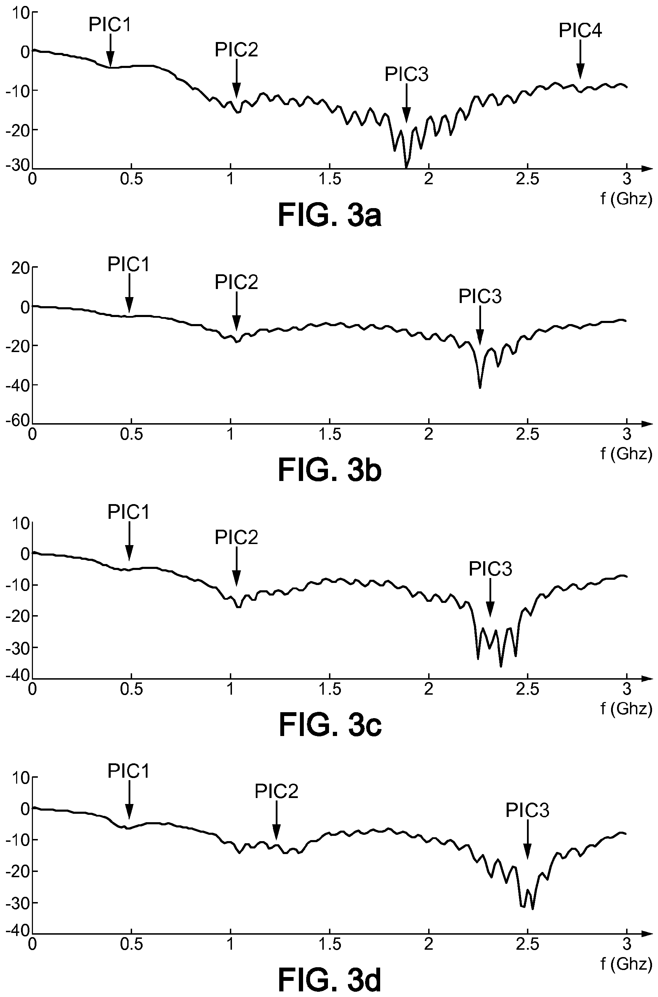

Les figures 3a à 3h sont issues de résultats expérimentaux et représentent les coefficients de réflexion du signal réfléchi SR en fonction d'une fréquence allant de 100 MHz à 3GHz respectivement :

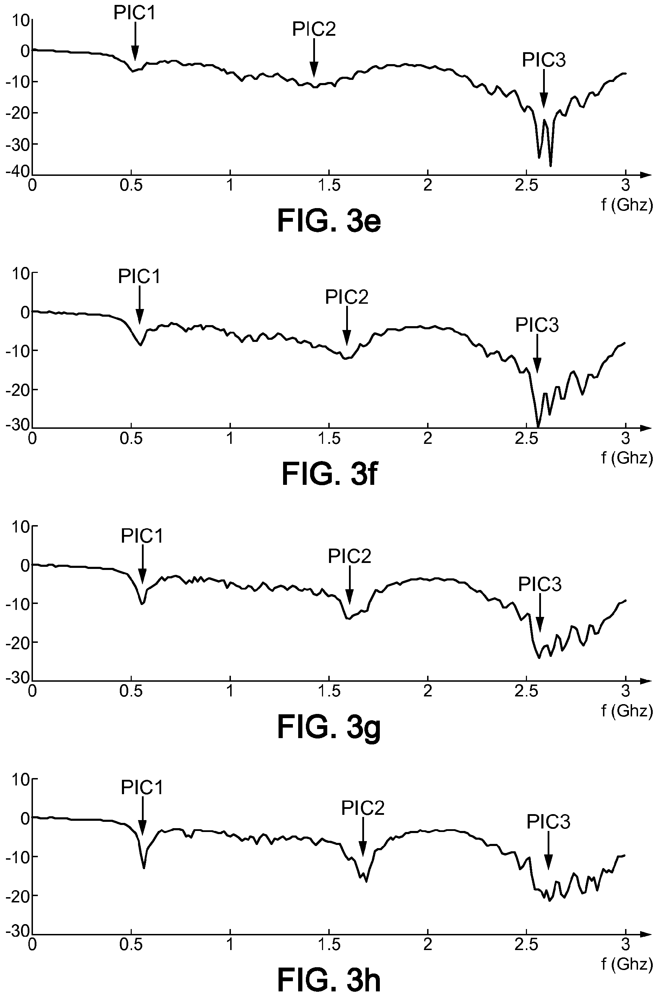

- le premier jour de séchage (figure 3a),
- le deuxième jour de séchage (figure 3b),
- le troisième jour de séchage (figure 3c),
- le cinquième jour de séchage (figure 3d),
- le dixième jour de séchage (figure 3e),
- le quinzième jour de séchage (figure 3f),
- le vingtième jour de séchage (figure 3g), et
- le trentième jour de séchage (figure 3h).

Comme illustré en figures 3a à 3h, il apparait dans le signal réfléchi SR un pic négatif pour les fréquences dans lesquelles le signal S_RF est « le mieux » transmis dans le mortier B.

Dans ces figures, on distingue un premier pic PIC1 autour 500MHz, un deuxième pic PIC2 autour de 1GHz et un troisième pic PIC3 autour de 2,3GHz.

On notera ici que, dans les premières heures (voir figure 3a), il est également possible de distinguer un quatrième pic PIC4 ; ce pic PIC4 disparaît ensuite.

Chacun de ces pics PIC1, PIC2, PIC3 et PIC4 correspond donc aux fréquences de résonance de l'antenne constituée par les segments 10a et 10a' dans le mortier B.

La fréquence de résonance dépend directement de la permittivité relative du matériau, et donc du taux d'humidité du matériau.

On observe en outre qu'en fonction du temps les trois pics PIC1, PIC2, PIC3 de fréquence de résonance se déplacent vers les hautes fréquences.

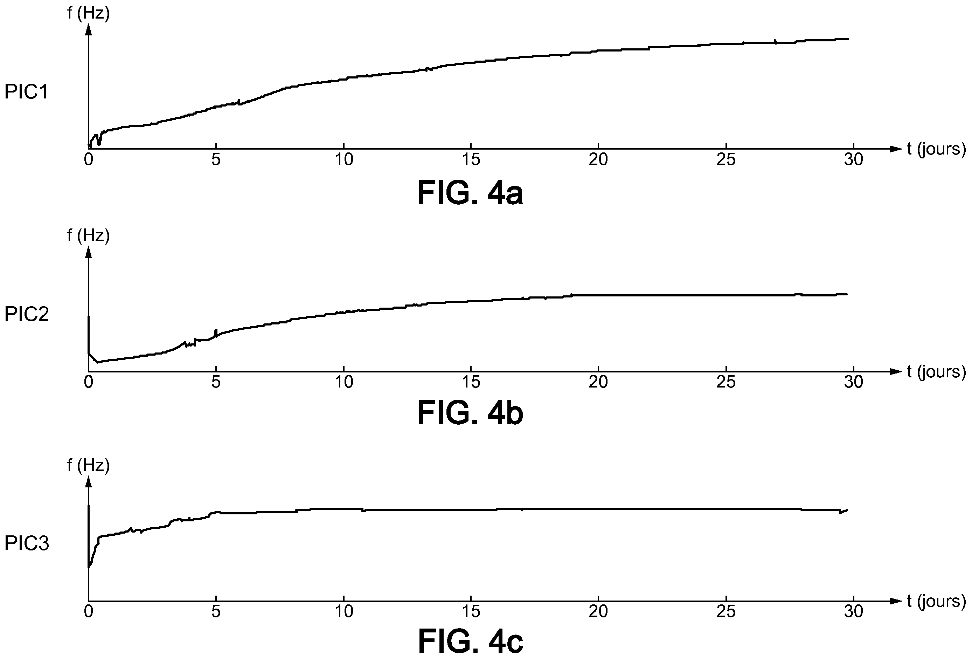

Les figures 4a, 4b et 4c exploitent les résultats ci-dessus et représentent l'évolution des fréquences de chaque pic PIC1, PIC2 et PIC3 (fréquence de résonance) pendant les 30 premiers jours.

Après analyse, l'évolution des fréquences de résonance est franche et suit très bien l'évolution de la teneur en eau dans le mortier au cours du séchage.

On remarque notamment sur ces figures 4a, 4b et 4c une rupture de chaque courbe après une dizaine d'heures de séchage : ce point de rupture correspond à une diminution de la quantité d'eau qui s'évapore.

En concomitance avec ce point de rupture, on observe également que chacune des fréquences marque un changement : l'amplitude et le sens de variation sont différents selon le pic observé.

Les courbes des figures 4a et 4b relatives aux deux premières fréquences de résonance observées (correspondant aux deux premiers pics) évoluent de façon continue et quasi similaire.

La courbe de la figure 4c qui est relative à la troisième fréquence de résonance observée (correspondant au troisième pic) met en évidence une variation forte les cinq premiers jours ; ensuite, cette courbe montre une stabilisation de cette fréquence.

Si l'on compare les résultats de ces mesures avec des résultats de mesures connus du type mesures du poids, on s'aperçoit que l'évolution de ces fréquences de résonance est inverse à l'évolution du poids du mortier.

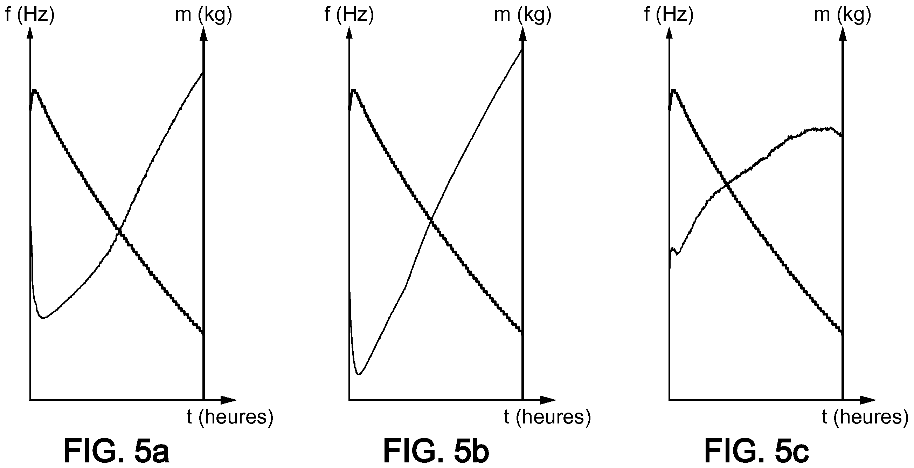

Ceci est confirmé par les graphiques des figures 5a, 5b et 5c qui représentent l'évolution de la fréquence de chacun des trois pics PIC1, PIC2 et PIC3 et l'évolution de la masse du mortier B pendant les premières heures de séchage.

L'analyse de ces graphiques permet de déduire qu'il existe un lien direct entre l'évolution de ces fréquences et l'évolution du poids.

L'analyse fréquentielle donne en plus une information très pertinente : les harmoniques des résonances du dipôle semblent en effet apporter des informations intéressantes sur la nature de l'eau présente dans le mortier.

Au début du séchage, il y a une forte évaporation d'eau jusqu'à la solidification du mortier (après deux à six heures de séchage).

Après quelques jours, l'eau est presque évaporée. Les fréquences du dernier pic ne changent quasiment plus après six jours de séchage.

Ainsi, les études menées ont permis d'observer que :

- les fréquences égales ou supérieures à 1,8 GHz donnent une information sur les changements d'eau de cristallisation ;
- les fréquences inférieures à 1,8 GHz donnent une information sur l'eau résiduelle (c'est-à-dire l'eau qui s'évapore du mortier).

Après calcul des coefficients de réflexion et détermination des fréquences de résonance, il est donc prévu une étape d'analyse fréquentielle S3 qui permet d'estimer avec précision le taux d'humidité du mortier B.

Cette analyse permet en outre de déterminer la nature de l'eau présente dans le mortier :

- eau résiduelle, ou
- eau de cristallisation.

Lorsque le taux d'humidité estimé lors de cette étape S3 atteint un taux seuil prédéterminé conforme aux normes en vigueur, alors l'opérateur peut couper l'interface 10b, 10b' dépassant de la chape de mortier B.

Ceci correspond à l'étape finale S4 de la mesure ; l'ouvrage peut se poursuivre.

Un premier avantage est que la mesure proposée ici dans le cadre de la présente invention est non destructive : il n'est donc plus nécessaire de prélever des échantillons dans le mortier.

De ce fait, les mesures réalisées ici selon la présente invention peuvent être multipliées pour suivre précisément l'évolution de la teneur en eau du mortier.

Un deuxième avantage est que le capteur 100 est simplement composé de fils 10 et 10', aussi fins que nécessaires, et donc qu'il n'est pas invasif tout en restant non local puisque la mesure se fait sur toute la longueur des fils 10 et 10' et notamment sur la longueur des segments 10a et 10a'.

Un troisième avantage est de pouvoir analyser très finement la nature de l'eau contenue dans le mortier B : la mesure proposée dans le cadre de la présente invention donne en plus des informations complémentaires sur la nature de l'eau contenue dans le mortier.

En effet, comme expliqué dans le préambule de la présente description, seule une partie de l'eau dans le mortier doit partir ; le reste fait partie de la composition du mortier sec ; ainsi, avec la mesure, on distingue dans le signal réfléchi :

- les fréquences égales ou supérieures à 1,8 GHz qui donnent une information sur les changements d'eau de cristallisation ;
- les fréquences inférieures à 1,8 GHz qui donnent une information sur l'eau résiduelle (c'est-à-dire l'eau qui s'évapore du mortier).

Un des nombreux autres avantages de la mesure proposée ici dans le cadre de la présente invention est d'avoir un temps de mesure extrêmement court : il suffit en effet à l'opérateur chargé de réaliser la mesure de brancher le réflectomètre 200 sur la partie du capteur 100 dépassant du mortier B et de lire sur l'appareil de mesure 100 les résultats sans effectuer de manipulations.

Cela permet, à temps d'intervention constante, de multiplier les mesures et d'optimiser l'avancement de la construction.

On remarquera également que, grâce à l'invention, il n'est plus nécessaire d'introduire dans le réflectomètre un résonateur.

On notera toutefois qu'un des désavantages de la présente invention est d'avoir une antenne perdue dans la chape de mortier B.

Cependant, l'antenne utilisée ici n'est composée que de fils 10 et 10' : son coût est donc très faible en comparaison du coût horaire d'un technicien devant effectuer une mesure du type mesure chimique utilisant du carbure de calcium.

On notera de plus que laisser l'antenne dans la chape de mortier B n'est pas défavorable du point de vue de la structure.

La présente invention met ainsi à disposition des acteurs de l'industrie de la construction, et particulièrement du bâtiment, une technologie simple d'utilisation pour mesurer et suivre l'évolution du taux d'humidité d'un matériau B tel que par exemple le mortier ou le béton en cours de séchage.

La technologie proposée ici nécessite par ailleurs peu de matériels : un simple système 300 composé d'un réflectomètre 200 et d'un capteur 100 instrumentant le matériau B à tester suffit.

Il devra être observé que cette description détaillée porte sur un exemple de réalisation particulier de la présente invention, mais qu'en aucun cas cette description ne revêt un quelconque caractère limitatif à l'objet de l'invention ; bien au contraire, elle a pour objectif d'ôter toute éventuelle imprécision ou toute mauvaise interprétation des revendications qui suivent.

**Revendications**

**1.** Procédé de mesure non-destructive du taux d'humidité d'un matériau (B), comprenant :

- l'émission (S1) par un appareil de mesure d'un signal radiofréquence (S_RF) dans le matériau (B) à travers une antenne (10a, 10a') noyée dans le matériau avec laquelle l'appareil de mesure coopère ;
- la réception (S2) par l'appareil de mesure d'un signal réfléchi (SR) par l'antenne en réponse au signal radiofréquence émis (S_RF) ;
- l'analyse (S3) en fréquence du signal réfléchi pour déterminer des fréquences de résonance de l'antenne (10a, 10a') dans le matériau (B) ;
- l'estimation du taux d'humidité du matériau (B) à partir des fréquences de résonance déterminées, l'antenne étant de type dipolaire.

**2.** Procédé selon la revendication 1, dans lequel le signal réfléchi (SR) est reçu et analysé en fréquence avec un déphasage nul par rapport au signal émis.

**3.** Procédé selon l'une quelconque des revendications précédentes, dans lequel l'analyse en fréquence du signal réfléchi comprend un calcul de coefficients de réflexion du signal réfléchi pour déterminer les fréquences de résonance.

**4.** Procédé selon l'une quelconque des revendications 1 à 3 pour mesurer le taux d'humidité d'un matériau solide (B) comprenant un ou plusieurs liants minéraux.

**5.** Procédé selon la revendication 4, dans lequel le matériau solide est un béton ou un mortier en cours de séchage.

**6.** Procédé selon la revendication 5, dans lequel, lorsque le matériau (B) est un mortier en cours de séchage, les fréquences de résonance supérieures ou égales à 1,8 GHz sont considérées comme caractérisant une présence d'eau de cristallisation dans ledit mortier, et les fréquences de résonance strictement inférieures à 1,8 GHz sont considérées comme caractérisant une présence d'eau résiduelle dans ledit mortier

**7.** Procédé selon l'une des revendications 4 à 6, dans lequel le matériau solide (B) est mis en œuvre comme élément de la structure de sol d'un local à usage d'habitation ou professionnel, pour la pose d'une couche de revêtement de sol sur ladite structure.

**8.** Procédé selon la revendication 7, dans lequel le matériau solide (B) est mis en œuvre sous la forme d'une dalle de béton ou d'une chape de mortier, ou encore d'une couche de ragréage.

**9.** Procédé selon l'une quelconque des revendications 1 à 3 pour mesurer le taux d'humidité d'un matériau (B) choisi parmi :

- un sol naturel,
- un matériau solide exempt de liant minéral et comprenant un ou plusieurs liants organiques,
- un matériau solide composite obtenu par frittage,
- un matériau pulvérulent,
- du bois ou
- un matériau liquide.

10. Système de mesure non-destructive pour mesurer un taux d'humidité d'un matériau (B), le système (100) comportant :

- un appareil de mesure (200) ;
- une antenne (10a, 10a') adaptée à être noyée dans le matériau (B) et coopérant avec l'appareil de mesure, l'antenne étant de type dipolaire ; et
- une interface entre l'antenne et l'appareil de mesure, l'appareil de mesure étant configuré pour :

- émettre un signal radiofréquence (S_RF) dans le matériau (B) à travers l'antenne (10a, 10a') ;
- recevoir un signal réfléchi (SR) par l'antenne en réponse au signal radiofréquence émis (S_RF) ;
- analyser en fréquence le signal réfléchi pour déterminer des fréquences de résonance de l'antenne (10a, 10a') dans le matériau (B) ;
- estimer le taux d'humidité du matériau (B) à partir des fréquences de résonance déterminées.

11. Système selon la revendication 10, dans lequel l'antenne est de type filaire et comprend deux segments (10a, 10a') qui s'étendent chacun dans le matériau (B) entre une extrémité proximale et une extrémité distale par rapport à l'interface avec l'appareil de mesure, les deux segments définissant des vecteurs ayant entre eux un produit scalaire négatif.

12. Système selon la revendication 11, dans lequel les deux segments s'étendent dans des directions opposées.

13. Système selon l'une quelconque des revendications 10 à 12, dans lequel l'antenne (10a, 10a') est isolée du matériau (B) par une gaine protectrice (20, 20'), de préférence constituée au moins partiellement en résine époxyde.

14. Système selon l'une quelconque des revendications 10 à 13, comportant un flotteur (30) amovible fixé à une portion (10b, 10b', 40) de l'interface dépassant du matériau (B) et configuré pour régler le positionnement de l'antenne dans le matériau (B), par exemple lors du coulage du matériau.

**Patentansprüche**

1. Verfahren zur zerstörungsfreien Messung des Feuchtigkeitsgehalts eines Materials (B), umfassend

- die Emission (SI) eines Radiofrequenzsignals (S_RF) durch eine Messvorrichtung in das Material (B) über eine in das Material eingebettete Antenne (10a, 10a'), mit welcher die Messvorrichtung zusammenwirkt;
- den Empfang (S2) eines von der Antenne als Antwort auf das ausgesendete Radiofrequenzsignal (S_RF) reflektierten Signals (SR) durch die Messvorrichtung;
- die Frequenzanalyse (S3) des reflektierten Signals, um die Resonanzfrequenzen der Antenne (10a, 10a') in dem Material (B) zu bestimmen;
- die Schätzung des Feuchtigkeitsgehalts des Materials (B) anhand der bestimmten Resonanzfrequenzen, wobei die Antenne von einem Dipoltyp ist.

2. Verfahren nach Anspruch 1, wobei das reflektierte Signal (SR) empfangen und mit einer Phasenverschiebung von Null gegenüber dem gesendeten Signal frequenzanalysiert wird.

3. Verfahren nach einem der vorstehenden Ansprüche, wobei die Frequenzanalyse des reflektierten Signals eine Berechnung von Reflexionskoeffizienten des reflektierten Signals umfasst, um die Resonanzfrequenzen zu bestimmen.

4. Verfahren nach einem der Ansprüche 1 bis 3 zum Messen des Feuchtigkeitsgehalts eines festen Materials (B), welches ein oder mehrere mineralische Bindemittel umfasst.

5. Verfahren nach Anspruch 4, wobei das feste Material ein trocknender Beton oder Mörtel ist.

6. Verfahren nach Anspruch 5, wobei, wenn das Material (B) ein trocknender Mörtel ist, Resonanzfrequenzen größer oder gleich 1,8 GHz als Anzeichen für das Vorhandensein von Kristallisationswasser in dem Mörtel angesehen werden und Resonanzfrequenzen, welche streng kleiner als 1,8 GHz sind, als Anzeichen für das Vorhandensein von Restwasser in dem Mörtel angesehen werden.

7. Verfahren nach einem der Ansprüche 4 bis 6, wobei das feste Material (B) als Element der Bodenkonstruktion eines Wohn- oder Geschäftsraums verwendet wird, um eine Bodenbelagsschicht auf dieser Konstruktion zu verlegen.

8. Verfahren nach Anspruch 7, wobei das feste Material (B) in Form einer Betonplatte oder eines Mörtelestrichs oder auch einer Ausgleichsschicht verwendet wird.

9. Verfahren nach einem der Ansprüche 1 bis 3 zum Messen des Feuchtigkeitsgehalts eines Materials (B), ausgewählt aus:

   - einem natürlichen Boden,
   - einem festen Material, welches frei von mineralischen Bindemitteln ist und ein oder mehrere organische Bindemittel enthält,
   - einem durch Sintern erhaltenen festen Verbundmaterial,
   - einem pulverförmigen Material,
   - Holz, oder
   - einem flüssigen Material.

10. Zerstörungsfreies Messsystem zum Messen eines Feuchtigkeitsgehalts eines Materials (B), wobei das System (100) umfasst:

    - eine Messvorrichtung (200);
    - eine Antenne (10a, 10a'), welche dazu eingerichtet ist, in das Material (B) eingebettet zu sein, und mit der Messvorrichtung zusammenwirkt, wobei die Antenne von einem Dipoltyp ist; und
    - eine Schnittstelle zwischen der Antenne und der Messvorrichtung, wobei die Messvorrichtung eingerichtet ist, zum:

      - Emittieren eines Radiofrequenzsignals (S_RF) über die Antenne (10a, 10a') in das Material (B);
      - Empfangen eines reflektierten Signals (SR) durch die Antenne als Antwort auf das ausgesendete Radiofrequenzsignal (S_RF);
      - Frequenzanalysieren des reflektierten Signals, um Resonanzfrequenzen der Antenne (10a, 10a') in dem Material (B) zu bestimmen;
      - Schätzen des Feuchtigkeitsgehalts des Materials (B) anhand der bestimmten Resonanzfrequenzen.

11. System nach Anspruch 10, wobei die Antenne von einem Drahttyp ist und zwei Segmente (10a, 10a') umfasst, welche sich jeweils in dem Material (B) zwischen einem proximalen Ende und einem distalen Ende in Bezug auf die Schnittstelle mit der Messvorrichtung erstrecken, wobei die beiden Segmente Vektoren definieren, welche untereinander ein negatives Skalarprodukt aufweisen.

12. System nach Anspruch 11, wobei sich die beiden Segmente in entgegengesetzte Richtungen erstrecken.

13. System nach einem der Ansprüche 10 bis 12, wobei die Antenne (10a, 10a') durch eine Schutzhülle (20, 20'), welche vorzugsweise zumindest teilweise aus Epoxidharz besteht, von dem Material (B) isoliert ist.

14. System nach einem der Ansprüche 10 bis 13, umfassend einen entfernbaren Schwimmer (30), welcher an einem Abschnitt (10b, 10b', 40) der Grenzfläche befestigt ist, welcher aus dem Material (B) herausragt, und welcher dazu eingerichtet ist, die Positionierung der Antenne in dem Material (B) einzustellen, beispielsweise beim Gießen des Materials.

**Claims**

1. A method for non-destructively measuring the moisture content of a material (B), comprising:

   - transmitting (S1) by a measurement apparatus a radio frequency signal (S_RF) in the material (B) through an antenna (10a, 10a') embedded in the material with which the measurement apparatus cooperates;
   - receiving (S2) by the measurement apparatus a signal (SR) reflected by the antenna in response to the radio frequency signal (S_RF) transmitted;
   - frequency-analysing (S3) the signal reflected to determine resonant frequencies of the antenna (10a, 10a') in the material (B);
   - estimating the moisture content of the material (B) from the resonant frequencies determined, the antenna being of the dipolar type.

2. The method according to claim 1, wherein the signal (SR) reflected is received and frequency-analysed with zero phase shift with respect to the signal transmitted.

3. The method according to any of the preceding claims, wherein frequency-analysing the signal reflected comprises calculating reflection coefficients of the signal reflected to determine the resonant frequencies.

4. The method according to any of claims 1 to 3 for measuring the moisture content of a solid material (B) comprising one or more mineral binders.

**5.** The method according to claim 4, wherein the solid material is concrete or mortar under drying.

**6.** The method according to claim 5, wherein, when the material (B) is mortar under drying, resonant frequencies greater than or equal to 1.8 GHz are considered as characterising presence of crystallisation water in said mortar, and resonant frequencies strictly less than 1.8 GHz are considered as characterising presence of residual water in said mortar.

**7.** The method according to one of claims 4 to 6, wherein the solid material (B) is implemented as an element of the floor structure of a room for residential or professional use, for laying a floor covering layer onto said structure.

**8.** The method according to claim 7, wherein the solid material (B) is implemented in the form of a concrete slab or a mortar screed, or a levelling layer.

**9.** The method according to any of claims 1 to 3 for measuring the moisture content of a material (B) selected from:

- a natural soil,
- a solid material free of mineral binder and comprising one or more organic binders,
- a solid composite material obtained by sintering,
- a powdery material,
- wood or
- a liquid material.

**10.** A non-destructive measurement system for measuring a moisture content of a material (B), the system (100) including:

- a measurement apparatus (200);
- an antenna (10a, 10a') adapted to be embedded in the material (B) and cooperating with the measurement apparatus, the antenna being of the dipolar type; and
- an interface between the antenna and the measurement apparatus, the measurement apparatus being configured to:

- transmit a radio frequency signal (S_RF) in the material (B) through the antenna (10a, 10a');
- receive a signal (SR) reflected by the antenna in response to the radio frequency signal (S_RF) transmitted;
- frequency-analyse the signal reflected to determine resonant frequencies of the antenna (10a, 10a') in the material (B);
- estimate the moisture content of the material (B) from the resonant frequencies determined.

**11.** The system according to claim 10, wherein the antenna is of the wire type and comprises two segments (10a, 10a') each extending in the material (B) between a proximal end and a distal end with respect to the interface with the measurement apparatus, the two segments defining vectors with a negative scalar product thereof.

**12.** The system according to claim 11, wherein the two segments extend in opposite directions.

**13.** The system according to any of claims 10 to 12, wherein the antenna (10a, 10a') is isolated from the material (B) by a protective sheath (20, 20'), preferably made at least partially of epoxy resin.

**14.** The system according to any of claims 10 to 13, including a removable float (30) attached to a portion (10b, 10b', 40) of the interface protruding from the material (B) and configured to adjust positioning of the antenna in the material (B), for example upon pouring the material.

**FIG. 1**

EP 3 268 735 B1

20,20'

10,10'

# FIG. 2

POSIT_ — S0

EMIT_ — S1

S_RF

RECEIV_ — S2

SR

ANALY_ — S3

THRES_ — S4

# FIG. 6

FIG. 3a

FIG. 3b

FIG. 3c

FIG. 3d

**FIG. 3e**

**FIG. 3f**

**FIG. 3g**

**FIG. 3h**

FIG. 4a

FIG. 4b

FIG. 4c

FIG. 5a

FIG. 5b

FIG. 5c

EP 3 268 735 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- DE 202013102514 U1

**Littérature non-brevet citée dans la description**

- **KUPFER K**. Radiofrequency and Microwave Moisture Sensing of Building Materials. *SENSORS UPDATE*, 01 January 2000, vol. 7, 27-50
- **ZBYSEK PAVLIK et al.** Application of Time-domain Reflectometry Method for measuring moisture content in porous building materials. *Trends in Applied Sciences Research*, 01 March 2007, vol. 2 (3), 188-200
- Hydration Monitoring and Moisture Control of Cement-Based Samples Through Embedded Wire-Like Sensing Elements. **CATALDO ANDREA et al.** IEEE SENSORS JOURNAL. IEEE, 01 February 2015, vol. 15, 1208-1215
- Optimization of a buried microstrip antenna for simultaneous communication and sensing of soil moisture. **SOONTORNPIPIT P et al.** IEEE TRANSACTIONS ON ANTENNAS AND PROPAGATION. IEEE, 01 March 2006, vol. 54, 797-800